# EUROPEAN PATENT APPLICATION

(11) **EP 1 782 765 A1**
(43) Date of publication of application: **09.05.2007**
(21) Application number: 06254735.1
(22) Date of filing: 12.09.2006
(51) Int. Cl.: A61F 2/40

(54) **Shoulder implant assembly**

(30) Priority: 23.09.2005 US 234743
(71) Applicant: Biomet Manufacturing Corp., Warsaw, IN 46581-0587 (US)
(72) Inventor: Schultz, Jason M., Hamilton, Ohio 45013 (US); Winslow, Nathan A., Warsaw, Indiana 46582 (US); Smeltzer, Dean E., Warsaw, Indiana 46580 (US); Stone, Kevin T., Winona Lake, Michigan 46590 (US)
(74) Representative: Giles, Ashley Simon

(57) **Abstract**

An implant assembly and associated method for selectively performing reverse and traditional arthroplasty for a shoulder joint that includes a humerus and a glenoid. The implant assembly may include a head, a cup, a humeral stem and an adaptor. The method includes inserting the humeral stem to the humerus and connecting a male taper of the adaptor to a female taper of the head. For reverse arthroplasty, the method includes attaching the adaptor to the glenoid and the cup to the stem. For traditional arthroplasty, the method includes attaching the adaptor to the humeral stem and the cup to the glenoid. The method also includes articulating the head with the cup.

## Description

### CROSS-REFERENCE TO RELATED APPLICATIONS

This application is a continuation-in-part application of United States Patent Application No. 10/680,924 filed on October 8, 2003. This application also claims the benefit of United States Patent Application No. 10/192,787, filed on July 10, 2002. The disclosures of the above applications are incorporated herein by reference.

### FIELD OF THE INVENTION

The present invention relates to an implant assembly for shoulder joint replacement.

### BACKGROUND OF THE INVENTION

A natural shoulder joint may undergo degenerative changes caused by a variety of reasons. When these degenerative changes become so far advanced and irreversible, it may ultimately become necessary to replace a natural shoulder joint with a prosthetic shoulder joint. In the traditional implantation of a shoulder joint prosthesis, the natural head portion of the humerus is resected and a cavity is created in the intramedullary canal of the host humerus for accepting a humeral component. The humeral component generally includes a stem, and a head portion, which is used to replace the natural head of the humerus. Once the humeral component has been implanted, the glenoid cavity positioned at the scapula may also be resected and shaped to accept a glenoid component. The glenoid component generally includes an articulating surface or cup which is engaged by the head portion of the humeral component. Modular designs for the humeral and glenoid components are currently available for the traditional shoulder arthroplasty, and components of different sizes or shapes are at the disposal of the surgeon performing the operation.

The traditional shoulder joint arthroplasty typically involves the coupling of a humeral head with a modified humerous, while a convcave bearing member can be placed on a prepared glenoid. In the reverse shoulder prosthesis, the humeral component includes a stem, and a cup connected to the stem. The glenoid component supports a head which articulates with the cup.

It is not always practical to determine well in advance of the procedure whether a reverse or traditional shoulder prosthesis should be used. It is, therefore, desirable to provide a selection of modular components that can be combined for use in traditional as well as reverse shoulder arthroplasty, with the goals of increasing flexibility and choice and for providing interchangeable and easy to use components that are also cost effective.

### SUMMARY OF THE INVENTION

One embodiment of the invention provides an implant assembly for a shoulder joint that has a humerus and a glenoid. The implant assembly may include a humeral stem configured to be inserted in the humerus, and a head bounded by a convex surface and a planar base that has a female taper. The implant assembly also includes an adaptor having a tray and a male taper. The tray may be configured to be attached to the glenoid. The male taper of the adaptor is configured to be received in the female taper of the head. The implant assembly also includes a cup that can be attached to the stem. The cup has a concave surface that is configured to articulate with the convex surface of the head.

Another embodiment of the invention provides an assembly of implant components for a shoulder joint having a humerus and a glenoid. The assembly includes a humeral stem, a head having a convex surface, and a cup configured to articulate with the convex surface of the head. The assembly optionally includes a glenoid adaptor for a reverse shoulder arthroplasty, and a humeral adaptor for traditional shoulder arthroplasty. The glenoid adaptor is configured to connect the head to the glenoid when the cup is connected to the humeral stem. The optional humeral adaptor is configured to connect the head to the humeral stem when the cup is connected to the glenoid. The same adaptor may be used as a glenoid and as a humeral adaptor.

Another embodiment of the invention provides an assembly of implant components for a shoulder joint having a humerus and a glenoid. The assembly includes a plurality of humeral stems, a plurality of heads, and a plurality of cups configured to articulate with the heads. The assembly also includes a plurality of glenoid adaptors for a reverse shoulder arthroplasty, and a plurality of humeral adaptors for traditional shoulder arthroplasty. The humeral or glenoid adaptors have an offset feature which allows for relative positioning of the humeral or glenoid articulating surfaces. Each glenoid adaptor is configured to connect one of the heads to the glenoid when one of the cups is connected to one of the humeral stems. Each humeral adaptor is configured to connect one of the heads to one of the humeral stems when one of the cups is connected to the glenoid.

Another embodiment provides a method for selectively performing reverse and traditional arthroplasty for a shoulder joint that includes a humerus and a glenoid. The method includes providing a head, a cup, and a humeral stem. The method also includes providing a humeral adaptor for traditional arthroplasty, and providing a glenoid adaptor for reverse arthroplasty. Further, the method includes selecting one of the humeral and glenoid adaptors, and performing the corresponding arthroplasty utilizing the head, the cup, the humeral stem and the selected adaptor.

Another embodiment provides a method for selectively performing reverse and traditional arthroplasty for a shoulder joint that includes a humerus and a glenoid. The method includes inserting a humeral stem to the humerus and connecting an adaptor to a head with mating male and female tapers. The method also includes selectively attaching a base of the adaptor to the glenoid for reverse arthroplasty, and to the stem for traditional arthroplasty, and selectively attaching a cup to the stem for reverse arthroplasty, and to the glenoid for traditional arthroplasty. Further, the method includes articulating the head with the cup.

Further areas of applicability of the present invention will become apparent from the detailed description provided hereinafter. It should be understood that the detailed description and specific examples, while indicating current embodiments of the invention, are intended for purposes of illustration only and are not intended to limit the scope of the invention.

### BRIEF DESCRIPTION OF THE DRAWINGS

The present invention will become more fully understood from the detailed description and the accompanying drawings. The components in the drawings are not necessarily to scale, emphasis instead being placed upon illustrating the principles of the invention.

Figure 1 is an exploded view of an embodiment of an implant assembly according to the invention, shown in a traditional shoulder arthroplasty;

Figure 2 is an exploded view of an embodiment of an implant assembly according to the invention, shown in a reverse shoulder arthroplasty;

Figures 3a-3d are side views of embodiments of an adaptor according to the present invention;

Figures 4a-4e are side views of embodiments of a cup according to the present invention; and

Figure 5a is an exploded view of an embodiment of an implant assembly according to the invention, shown in a traditional shoulder arthroplasty and with alternative heads and humeral adaptors;

Figure 5b is an exploded view of an embodiment of an implant assembly according to the invention, shown in a reverse shoulder arthroplasty;

Figure 6a is an exploded view of an embodiment of an implant assembly according to the invention, shown in a traditional shoulder arthroplasty;

Figure 6b is an exploded view of an embodiment of an implant assembly according to the invention, shown in a reverse shoulder arthroplasty;

Figure 7 is an exploded view of an embodiment of an implant assembly according to the invention, shown in a reverse shoulder arthroplasty;

Figure 8 is an embodiment of a assembly of components for shoulder arthroplasty according to the invention;

Figures 9a-9c are side views of embodiments of heads according to the invention;

Figures 10a-10c are side views of embodiments of adaptors corresponding to the heads of Figures 9a-9c;

Figures 11 a-11 c are side views of embodiments of head bearings;

Figure 12 is an exploded view of an embodiment of an implant assembly according to the invention, shown in a traditional shoulder arthroplasty;

Figure 13 is an exploded view of an embodiment of an implant assembly according to the invention, shown in a traditional shoulder arthroplasty without a humeral stem;

Figure 14 is an exploded view of an embodiment of an implant assembly according to the invention, shown in a reverse shoulder arthroplasty without a humeral stem;

Figure 15 is an exploded view of an embodiment of modular adaptor, shown with glenoid and humeral stems;

Figure 16 is an exploded view of an embodiment of modular adaptor; shown with glenoid and humeral stems;

Figure 17 represents a perspective exploded view of an alternate shoulder prosthetic;

Figures 18a - 18c represent side views of the shoulder prosthetic shown in Figure 17;

Figures 19a and 19b represent side views of an alternate shoulder assembly;

Figures 20a and 20b represent side views of an alternate shoulder assembly;

Figures 21 a and 21 b represent side views of an alternate shoulder assembly;

Figures 22a and 22b represent side views of an alternate shoulder assembly;

Figures 23a and 23b depict the assembly of a humeral component shown in the joint prosthetic shown in Figured 20a and 20b;

Figures 24a to 26b represent views of the glenoid component shown in Figures 17-21 b;

Figures 27a to 29b represent bearings used in the assemblies shown in Figures 18a, 18b, 20a and 20b;

Figures 30a-32 represent head components shown in Figures 17-22b;

Figures 33a-33c represent the adaptor shown in Figures 17-23b;

Figure 34 represents a kit of components shown in Figures 17-33;

Figure 35 represents the humeral implant for a reverse shoulder prosthetic;

Figures 36-40 represent an implant shown in Figure 35 utilizing an alternate head;

Figures 41 a and 41 b represent glenoid fixation components; and

Figure 42a and 42b represent a humeral body portion shown in Figures 35-40.

### DETAILED DESCRIPTION OF THE PREFERRED EMBODIMENTS

The following description of the preferred embodiments of the invention is merely exemplary in nature and is in no way intended to limit the invention, its application, or uses.

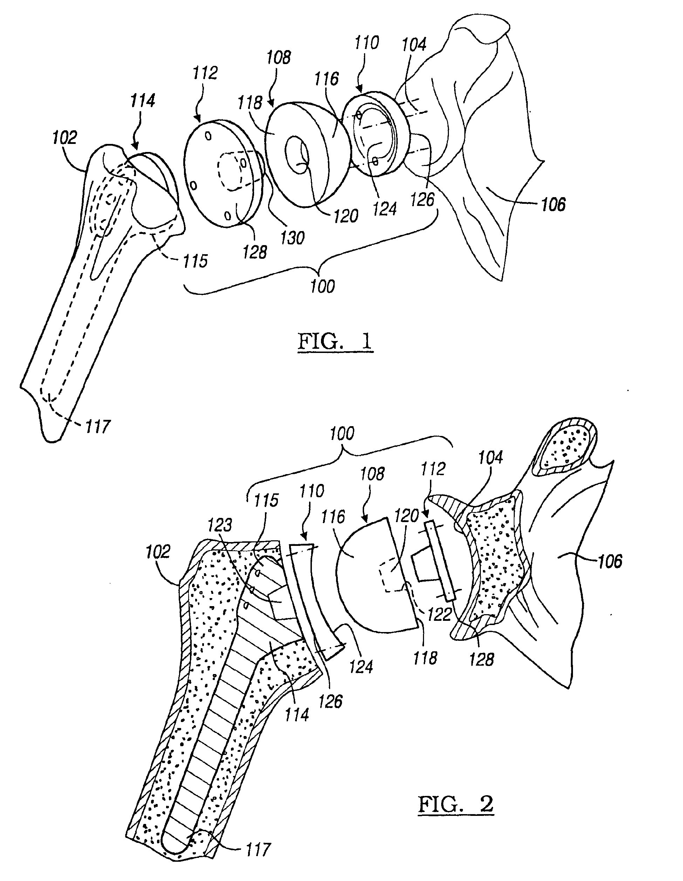

Referring to Figures 1 and 2, there is shown an embodiment of an implant assembly 100 for a total shoulder joint replacement. The implant assembly 100 is configured to be implanted between a resected humerus 102 and a glenoid cavity ("glenoid") 104 of a scapula 106 in one of two ways, i.e., in a traditional arthroplasty depicted in Figure 1, or in a reverse arthroplasty depicted in Figure 2, by selecting and/or reconfiguring appropriately the components of the implant assembly 100. The implant assembly 100 includes a head 108, a cup 110, and an adaptor 112. The implant assembly 100 may also include a humeral stem 114 that has a proximal end 115 and a distal end 117.

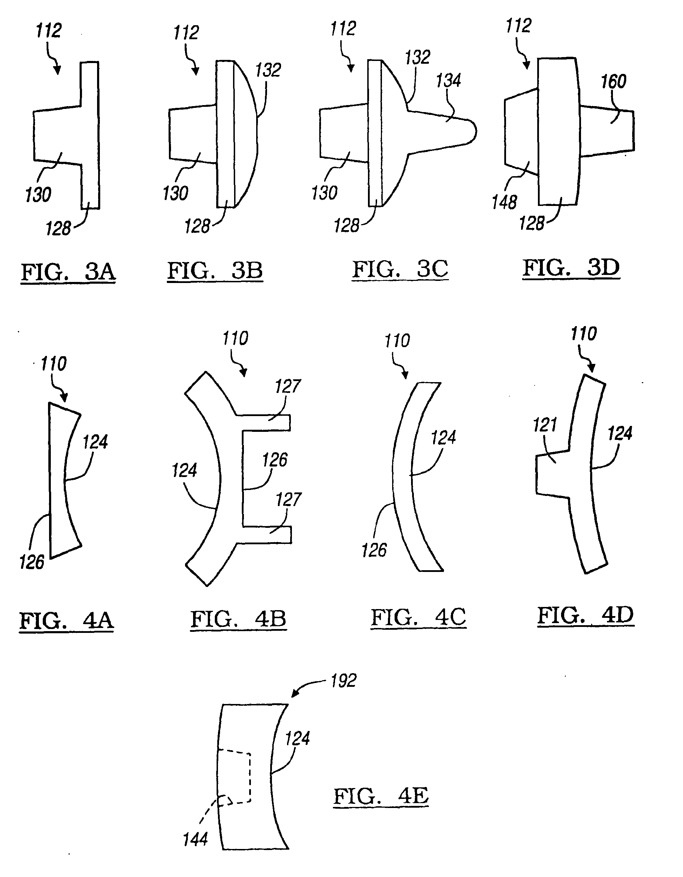

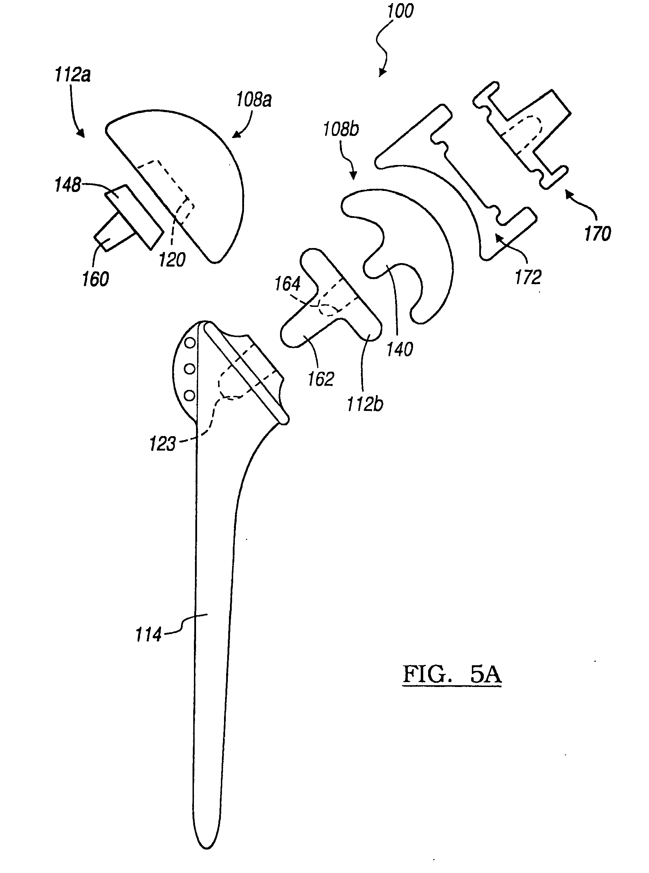

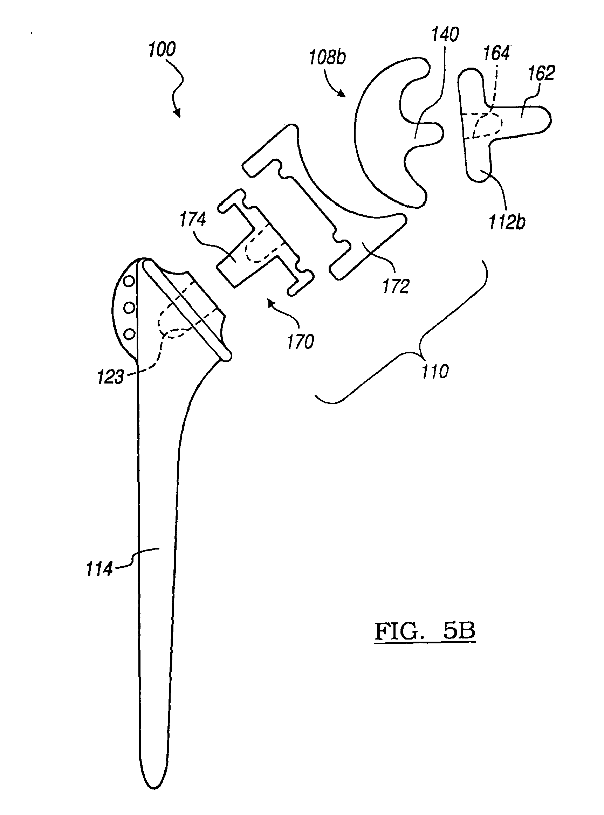

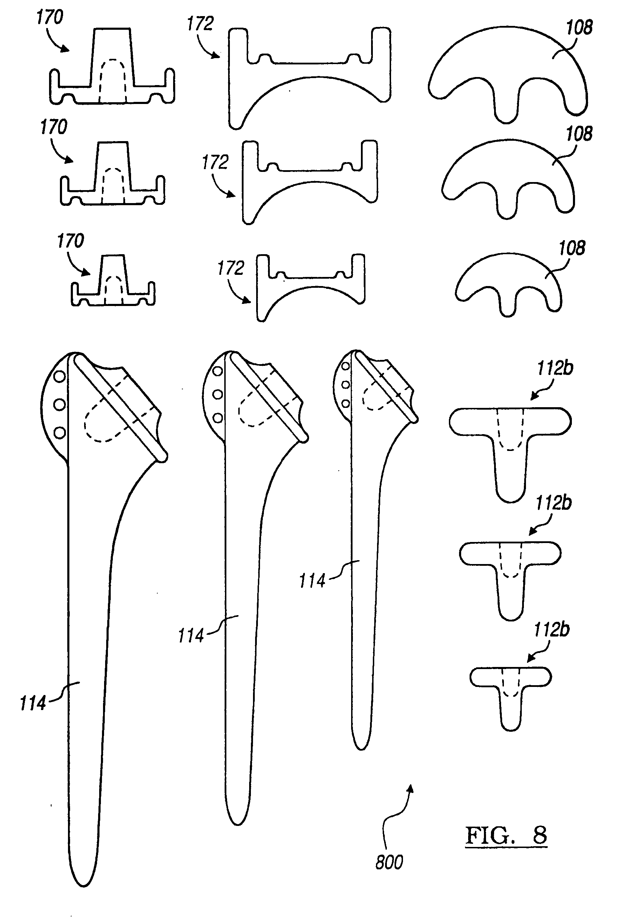

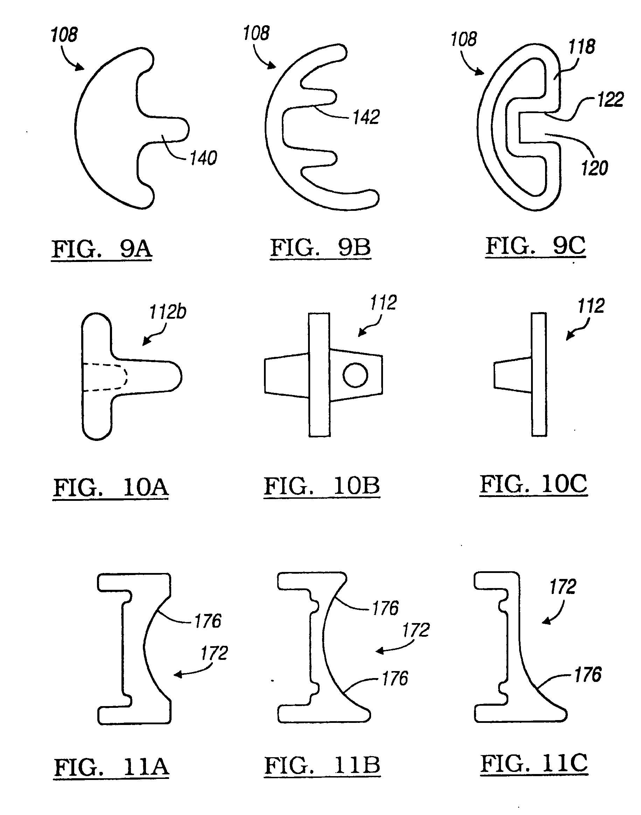

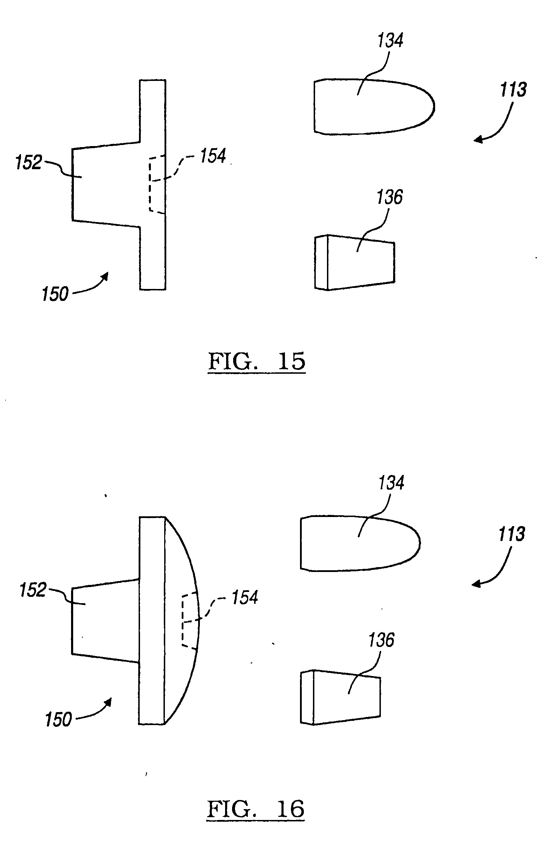

Other embodiments of the implant assembly are shown in Figures 5a, 6a, 12, 13 for traditional shoulder replacement, and in Figures 5b, 6b, 7 and 14 for reverse shoulder replacement. Figures 3a-3d, 4a-4e, 9a-9c, 10a-10c, 11 a-11 c show representative embodiments of various components. Figures 15 and 16 show embodiments of a modular adaptor 113. Figure 8 shows an embodiment of a component assembly (kit) for shoulder replacement 800 showing different sizes of representative components. It should be understood that the component assembly 800 in Figure 8 is only illustrative of the inclusion of different sizes of each component and it is not limited by the type of components actually shown. For example, the component assembly 800 may include different sizes of each of the heads 108 shown in Figures 9a-9c, different types and sizes of adaptors 112, different sizes and types of cups 110, etc. Like reference numerals refer to like components. When clarity requires differentiating between different embodiments of the same component, an alphabetic character is attached to the reference numeral. For example, the head 108 is referenced as head 108a and head 1 08b to distinguish between two different head embodiments, as shown in Figure 5a.

In the embodiment shown in Figures 1 and 2, the head 108 is bounded by a convex surface 116, which may be, for example, a hemispherical surface, and a base 118, which may be a substantially planar surface. In one embodiment, the base 118 may be modularly connected to the head 108. A female taper 120 with tapered inner walls 122 extends from the base 118 into the head 108. The convex surface 116 of the head 108 is shaped to articulate with a concave surface 124 of the cup 110 to allow for shoulder joint movement. Such articulation may be centered or eccentric. This and other embodiments of the head 108 are shown in Figures 9a-9c. In the embodiment of Figure 9a, the head 108 has a male taper 140. In the embodiment of Figure 9b, the head 108 has a female taper 142.

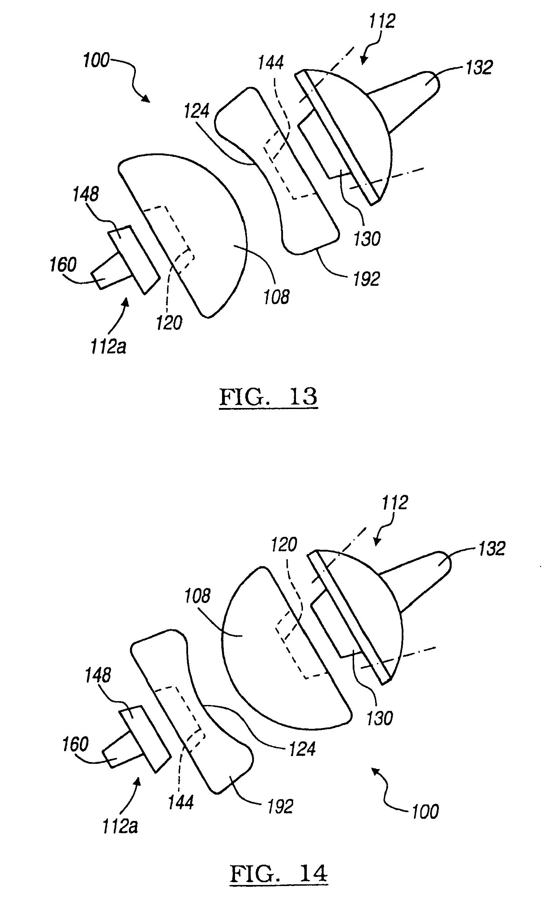

Referring to Figures 1 and 2, the cup 110 may include a back surface 126 that may be configured to be selectively attached to the humeral stem 114 in reverse shoulder arthroplasty, or to the resected glenoid 104 in the traditional shoulder arthroplasty. Alternatively, the cup 110 may be chosen from a number for available cups, such as those shown in Figures 4a-4d, some of which are better suited to either reverse shoulder arthroplasty or traditional shoulder arthroplasty. For example, the back surface 126 of the cup 110 may be a substantially planar surface which can be attached with cement or with mechanical fasteners, such as screws, to the humeral stem 114 or to the appropriately resected glenoid 104. Alternatively, as shown in Figure 4c, the back surface 126 may be slightly convex. In the embodiment shown in Figure 4b, the back surface 126 may include a number of pegs 127 for attachment to the glenoid 104. In the embodiment shown in Figure 4d, the cup 110 may include a male taper 121 which is received in a corresponding female taper 123 of the proximal end 115 of the humeral stem 114. Figure 4e shows a bearing 192 of a modular cup 110 that has a concave surface 124 and a female taper 144 adapted to receive a modular glenoid stem 130 or modular humeral stem 136, such as those shown in Figures 15 and 16 in connection with the modular adaptor 113. The bearing 192 may also be used with the embodiments of Figures 13 and 14, as is described below.

Referring to Figures 15 and 16, the modular adaptor 113 may include a body 150 with a male taper 152 and a female taper 154. The female taper 154 is adapted to receive a glenoid stem 134 for the reverse shoulder arthroplasty and a humeral stem 136 for the traditional shoulder arthroplasty. The male taper 152 is adapted to be received in the female taper 142 of the embodiment of the head 108 shown in Figure 9b or in the female taper 120 of the embodiment of the head 108 shown in FIGURE 9c, for example.

In general, the adaptor 112 may be modular, such as the adaptor 113 of Figures 15 and 16, or a monolithic adaptor. The adaptor 112 may be a single, one and the same, adaptor that can be used selectively in both the traditional and the reverse shoulder arthroplasty, or it can be chosen from a number of available adaptors of an assembly of components, such as those shown in Figures 3a-3d, depending on which arthroplasty procedure is to be performed. Some of these adaptors 112, such as, for example, the adaptor shown in Figure 3c, may be specifically configured for use with reverse arthroplasty, because they incorporate the glenoid stem 134, either modularly or monolithically.

In one embodiment, the adaptor 112 may include an adaptor tray 128 and an extension or male taper 130 that can be press-fitted into the female taper 120 of the head 108. For the procedure of traditional shoulder arthroplasty, the tray 128 is attached to the proximal end of the humeral stem 114, as shown in Figure 1. For the procedure of reverse arthroplasty, the tray 128 is attached to the glenoid 104, as shown in Figure 2. In the embodiments of Figures 3a and 3c, the tray 128 may include a curved portion 132 shaped to conform to a portion of the glenoid 104. It may also include the glenoid stem 134, which is inserted into the glenoid 104. The adaptor may be attached to the glenoid 104 with fasteners, such as screws.

Referring to the embodiment of Figure 3a, the tray 128 may also be substantially planar. It will be appreciated, however, that other shapes, in addition to those shown in Figures 3a-3d, are possible for the tray 128 depending, for example, on the various ways the tray 128 is be attached to the humerus 102, to the humeral stem 114, or to the glenoid 104. Furthermore, the adaptor 112 may be modular, such that the male taper 130, the tray 128 and the glenoid stem 134 are all separate components interconnected though fasteners, such as screws, or other type of connectors, including male-female tapers as illustrated in Figures 15 and 16. The adaptors 112, 112a shown in Figures 3d and 5a include a male taper 148 which can be received in the female taper of 120 of the head 108, 108a, and a male taper 160 which can be received in the female taper 123 of the humeral stem 114.

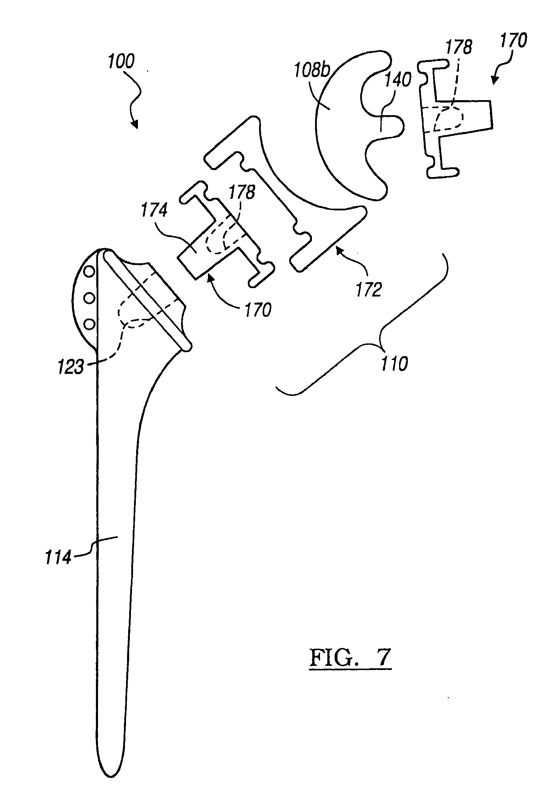

In the embodiments illustrated in Figures 5b and 7, the cup 110 may be replaced by a bearing base 170, which is also an adaptor, and a bearing 172 that can be fitted to the base 170. The bearing base 170 has a male taper 174 configured to be received in the female taper 123 of the humeral stem 114. Examples of bearings 172 with symmetric or non-symmetric and eccentric bearing surfaces 176 are shown in Figures 11 a-11 c. In the embodiment of Figure 7, the bearing base 170, includes a female taper 178 adapted to receive the male taper 140 of the head 108b.

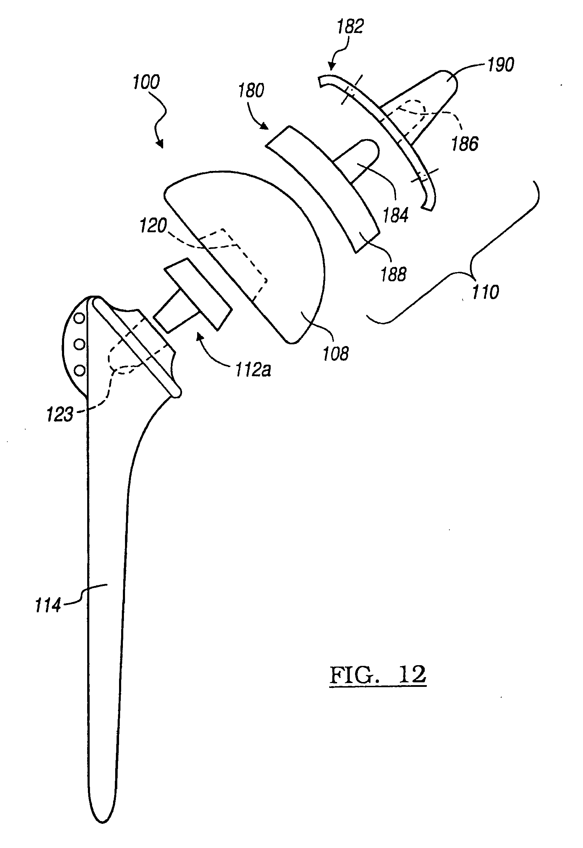

In the embodiment of Figure 12, the cup 110 may be replaced by a bearing 180 that is fitted in a bearing base 182 with mating male taper 184 and female taper 186, or with a bearing 180 that includes only a liner 188. The bearing base 182 may include a modular or integral glenoid stem 190.

Referring to Figures 1 and 2, for example, the implant assembly 100 may be used as follows. The humeral stem 114 is inserted in the resected humerus 102. The adaptor 112 is attached to the head 108 by inserting the male taper 130 into the female taper 120. For traditional shoulder arthroplasty, the cup 110 is attached to the glenoid 104, and the adaptor 112 is attached to the proximal end 115 of the humeral stem 114, such that the convex surface 116 of head 108 articulates with the concave surface 124 of the cup 110. For reverse shoulder arthroplasty, the cup 110 is attached to the proximal end 115 of the stem humeral 114 and the adaptor 112 is attached to the resected glenoid 104 such that the convex surface 116 of head 108 articulates with the concave surface 124 of the cup 110. Although the same adaptor 112 can be used for both the traditional and the reverse shoulder arthroplasty procedures, glenoid-specific adaptors 112 may be chosen, either as integral components or built from modular parts that include male tapers 130, trays 128 and glenoid stems 134.

It will be appreciated that the individual components of the implant assembly 100 may be made using a variety of materials, including metal and plastic. The head and the stem may be made of metallic material, such as a cobalt chrome alloy, for example. Porous coating may be provided for the proximal end of the stem. The cup may be made of polyethylene or metal or a combination thereof, such as polyethylene bearing or lining and metal base. The adaptor can be typically made of metal.

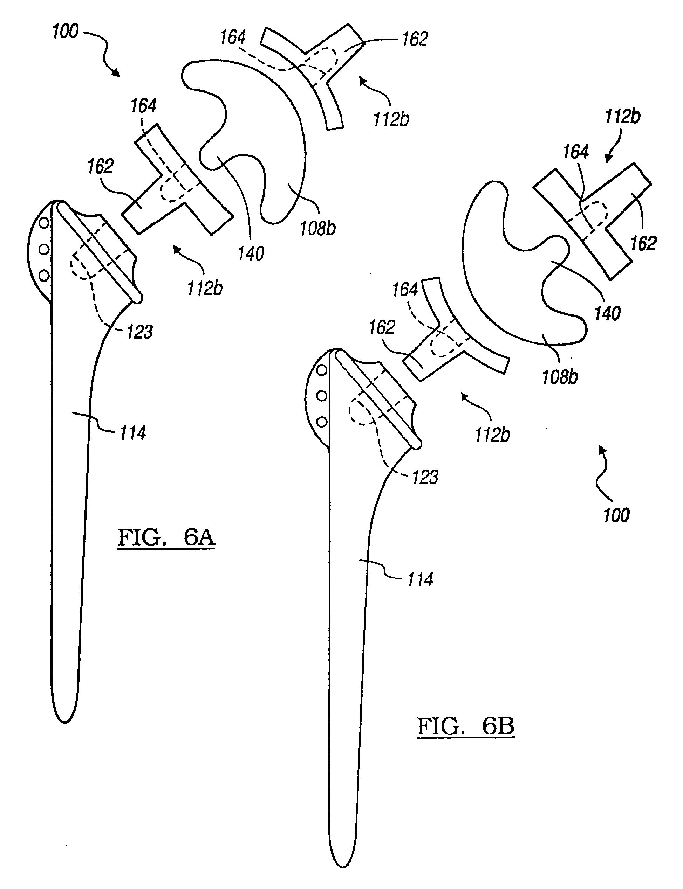

Other exemplary embodiments are illustrated in Figures 5a, 5b, 6a, 6b, 7 , and 12-14. In Figure 5a, the male taper 140 of the head 108b can also be inserted directly into the female taper 123 of the humeral stem 123. Alternatively, an adaptor 112b having a male taper 162 and a female taper 164 may be provided. The adaptor 112b may be also used in the embodiments shown in Figures 5b, 6a, and 6b.

In the embodiments of Figures 13 and 14, the male taper 148 of the adaptor 112a can be received in the female taper 120 of the head 108 for the traditional shoulder arthroplasty shown in Figure 13, and in the female taper 144 of the bearing 192 for the reverse arthroplasty shown in Figure 14. Similarly, the male taper 130 of the glenoid adaptor 112 may be received in the female taper 120 of the head 108 for the reverse shoulder arthroplasty, and in the female taper 144 of the bearing 192 for the traditional shoulder arthroplasty.

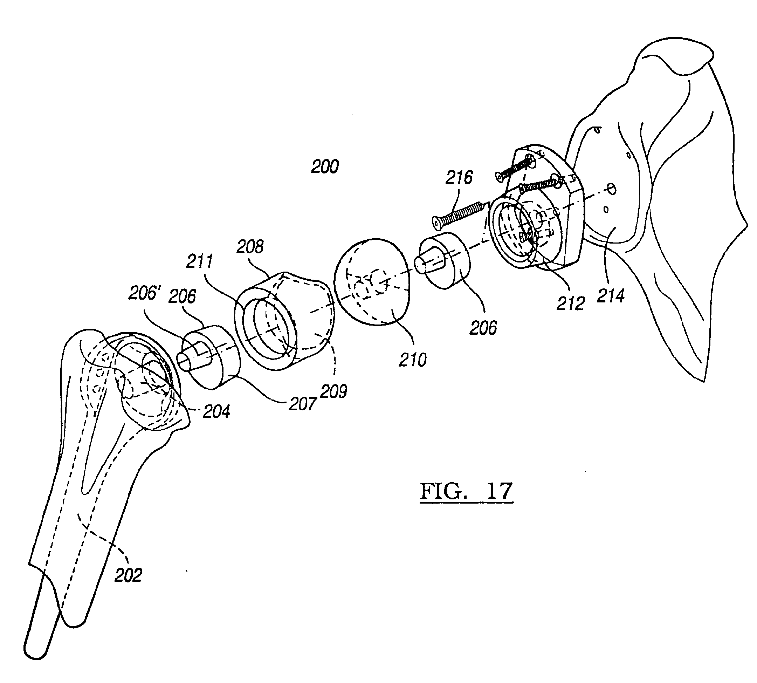

Figure 17 represents a perspective exploded view of an alternate shoulder prosthetic 200. The prosthetic 200 has a humeral stem 202 which is mated to a bearing 208 that interfaces with a head portion 210. The head portion 210 is coupled to a prepared glenoid 214. The humeral stem 202 has a coupling portion 204 which is configured to mate with a coupling taper 206' on adapter 206. The adaptor 206 has a coupling taper or taper lock connection 207 which is configured to couple to a corresponding coupling taper 211 disposed on a surface of the bearing 208. The bearing 208 has a bearing surface 209 which articulates with the articulating surface of the head 210.

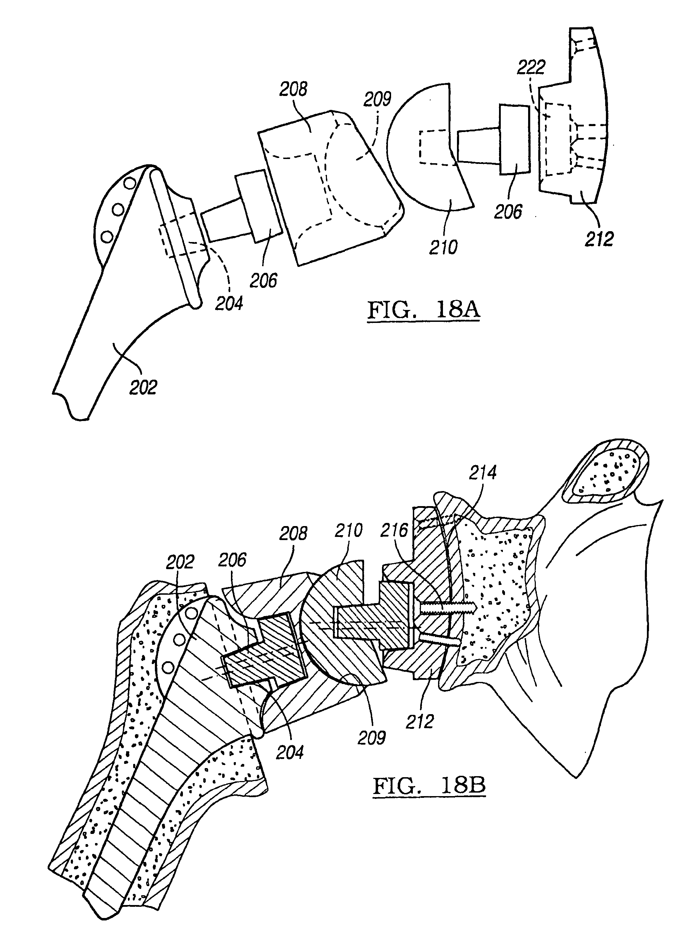

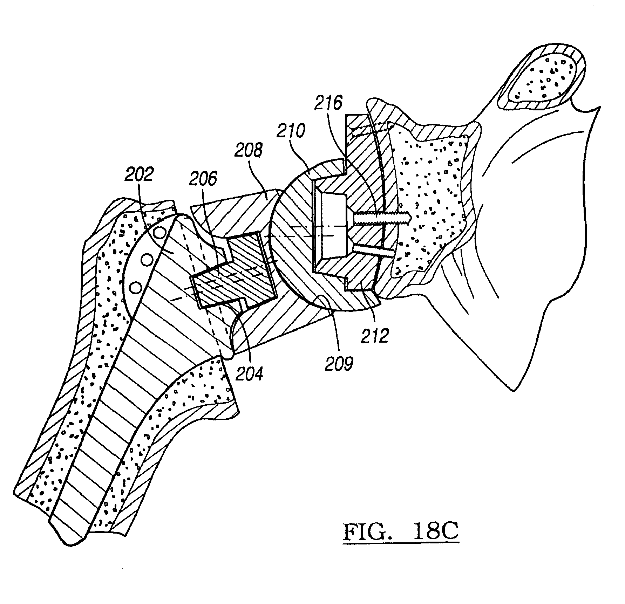

Figures 18a and 18b represent a side view of the prosthetic 200 shown in Figure 17. A second adaptor 206 is disposed between head 210 and the glenoid component 212. The adaptor 206 is configured to interface with a coupling taper 222 defined within the glenoid bearing member 212. It is envisioned the coupling taper 222 can be either a male or a female taper lock connection configured to mate with an appropriate taper on the head 210 (see Figure 18c).

As shown in Figure 18b, the glenoid bearing member 212 is coupled to the prepared glenoid 214 using a plurality of fixation screws 216. The adaptors 206 optionally can have a pair of locking tapered members which are off axis from each other, allowing a physician to rotate the offset to alignment the components within the joint to increase the range of motion of the prosthetic 200. In this regard, the rotation of the adaptors 206 allows for the radial, rotational and angular positioning of the head and cup members. Further it is envisioned that optionally, at least some of the components can be used in a traditional arthroplasty.

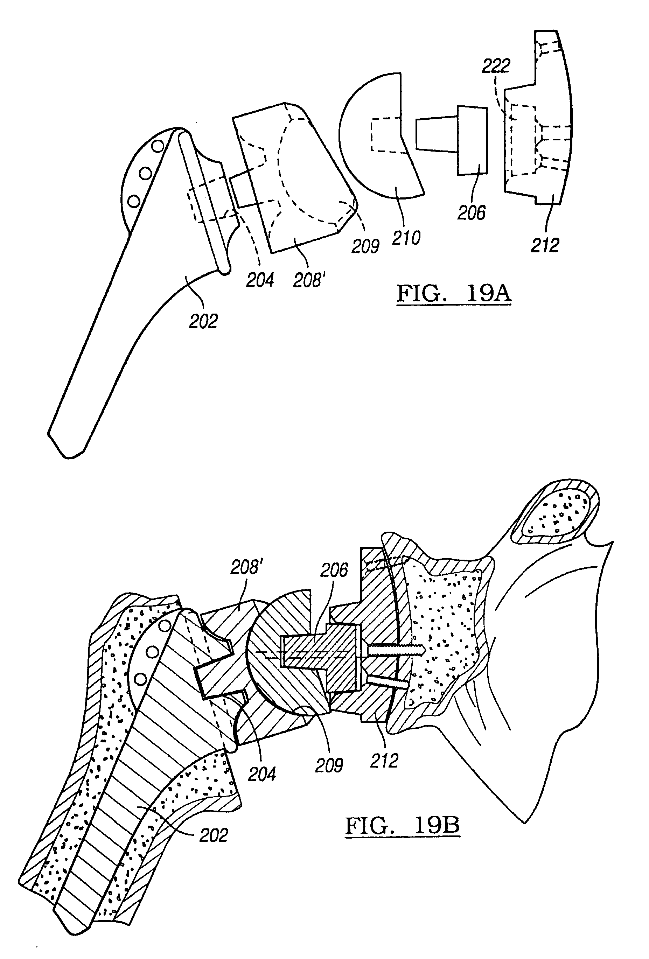

As shown in Figures 19a and 19b, the bearing member 208' can be coupled directly to the coupling portion 204 of the humeral stem 202. In this regard, the bearing 208' can have a male coupled taper 206 or a female coupling taper. As shown in Figure 19b, the use of an adaptor 206 having an offset tapered stem can allow for relative movement of the articulating head 210 with respect to the bearing 208.

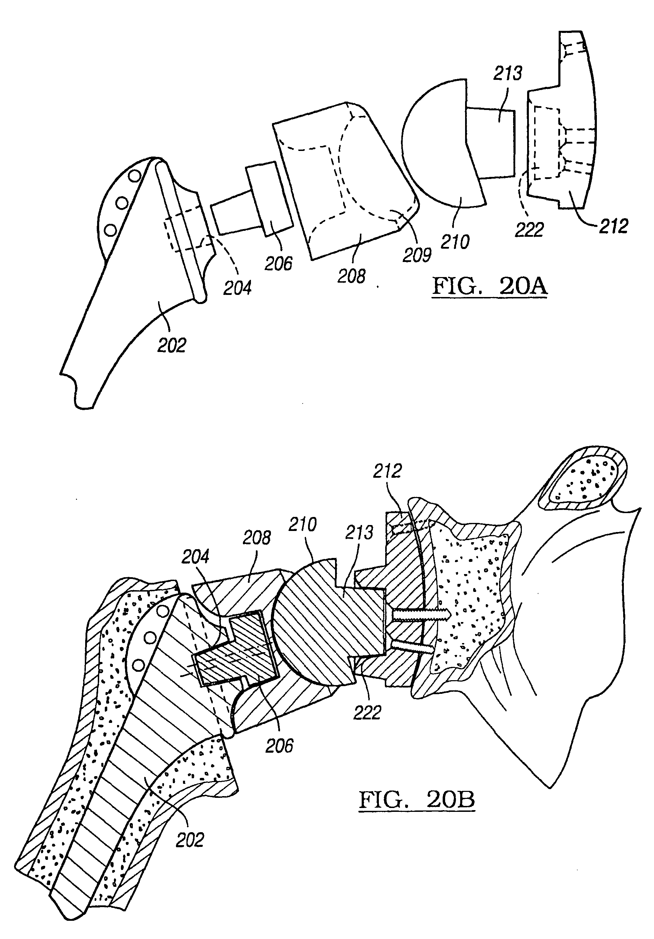

As shown in Figures 20a and 20b, the articulating head 210 can have a stem 213 which is configured to couple with a female locking taper 222 within the glenoid component 212. The use of a offset adaptor 206 located between the bearing 208 and the coupling portion 204 of the humeral stem 202 allows for relative displacement of the bearing surface 209 of the bearing member 208 with respect to the head portion 210. Furthermore, by removing the adaptor 206, the size of the joint can be reduced.

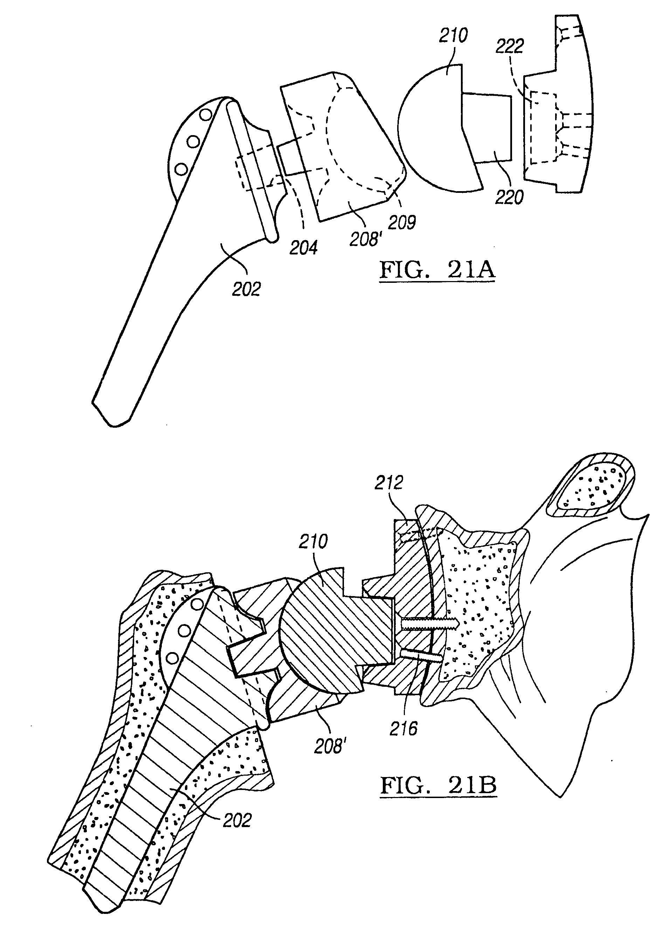

As shown in Figures 21 a and 21 b, the bearing 208 can be directly coupled to a coupling portion 204 of the stem 202. Additionally, the head 210 can be coupled directly to the glenoid component 212 to reduce the overall size of the joint. The direct coupling of the components is accomplished by using locking tapers or fixation members such as threaded fasteners or adhesive.

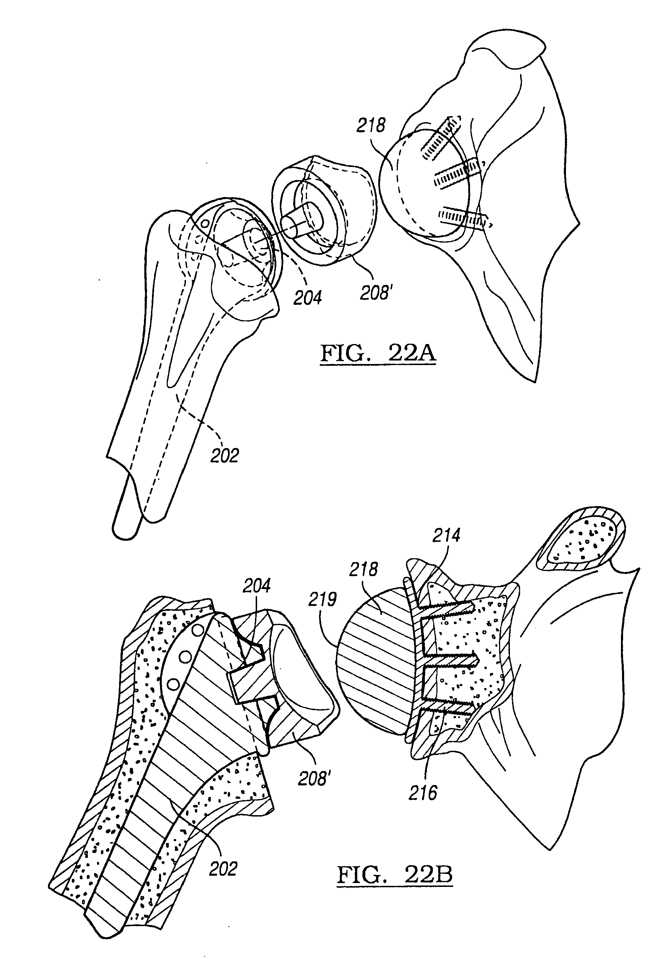

As shown in Figures 22a and 22b, a head 218 can be directly coupled to the glenoid 214. This head 218 can be directly coupled to the glenoid 214 using a plurality of bone fixation screws 216 coupled to the head 218. Additionally, an attachment tray (not shown) can be used to couple the head to the prepared glenoid. The bearing surface 219 of the head 218 can have a varying radius of curvature over its surface. In this regard, the radius of curvature can be specifically design to interface with the bearing surface 209 of the bearing member 208 to increase the range of motion while reducing chances of dislocation of the joint.

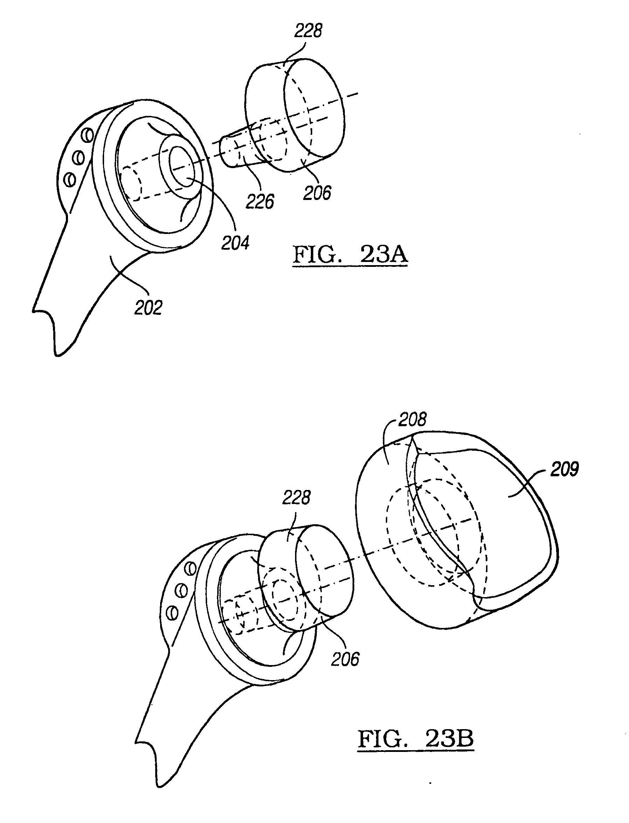

Figures 23a and 23b represent the coupling of the bearing member to the humeral component 202. The adaptor 206 is coupled to coupling portion 204 of the stem. In this regard, a male taper lock connection 226 is disposed within the coupling portion 204. The bearing 208 is then coupled to a male taper lock connection 228 disposed on the adaptor 226. A trialing adaptor 206 can be used to allow the placement of the bearing member 208. In this regard, the trailing head is non-fixably coupled to the stem and rotated to place the cup in its proper location. At this point, a regular adaptor 206 is fixably coupled the stem using a impactor as is know.

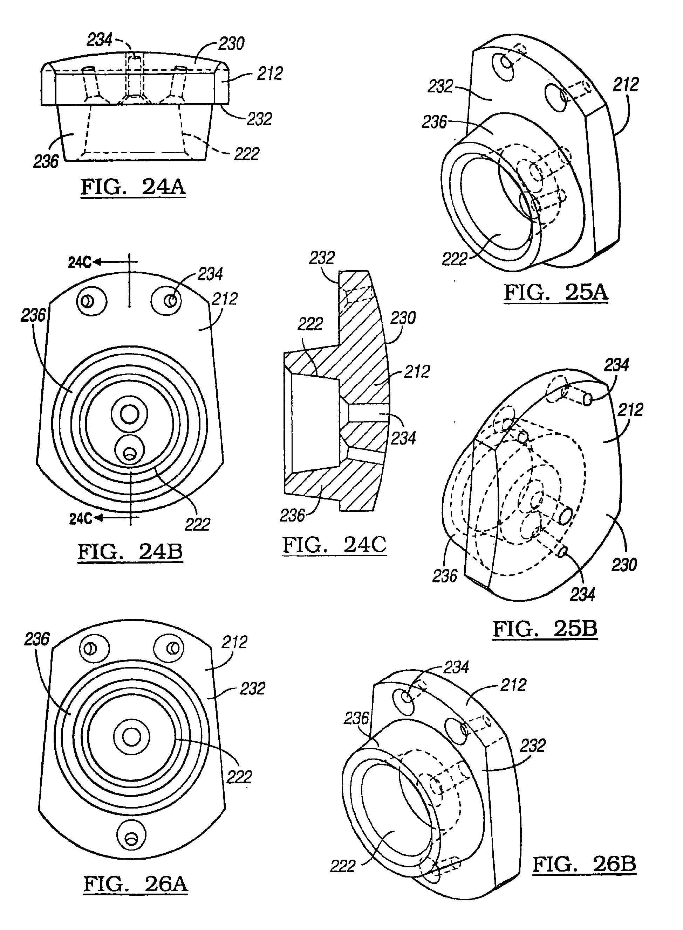

Figures 24a-26b represent alternate views of the glenoid member 212. The glenoid member 212 has a first curved coupling surface 230 which is configured to be mated to a curved surface on the prepared glenoid 214. Additionally, the glenoid 212 has a outward facing surface 232 which is generally opposite to the coupling surface 230. Disposed on the outward surface 232 is a boss portion 236 which defines an exterior fixation taper. Additionally, the boss 236 defines the interior taper 222 which is configured to fixedly accept a male taper of the adaptor 206. Defined through the glenoid component is a plurality of bone fixation holes 234. The bone fixation holes 234 are angled with respect to each other to provide enhanced fixation of the glenoid member 212 to the prepared glenoid 214.

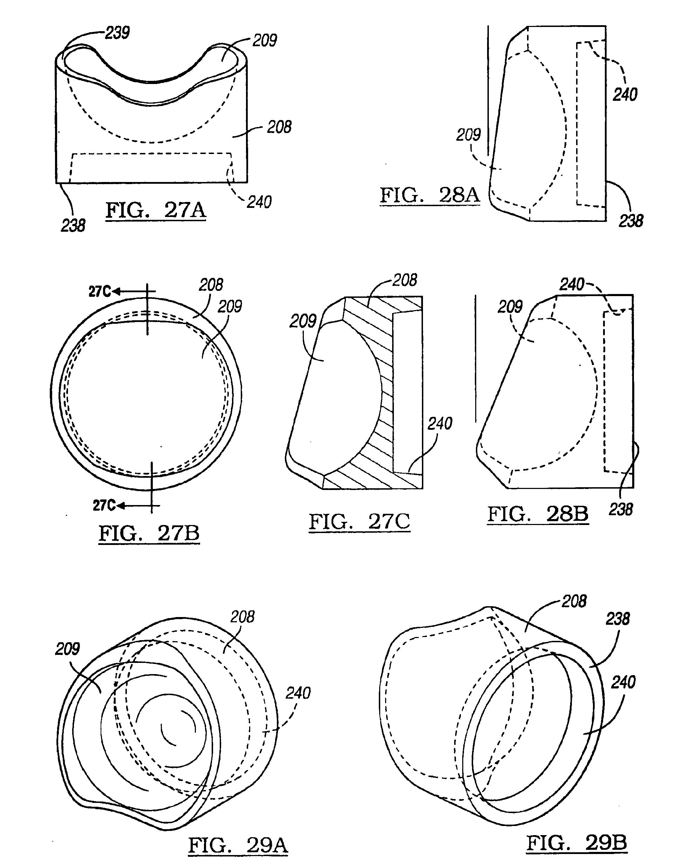

Figures 27a-29b represent alternate bearing members 208. Disposed on a coupling surface 238 is the coupling taper 240. The coupling taper 240 is configured to be mated either with an adaptor 206 or with the humeral fixation member 204. Defined on a bearing side 239 of the adaptor 208 is the bearing surface 209. The bearing surface 209 can vary in curvature to maximize the articulation of the head on the bearing surface 208 while minimizing the possibility of dislocation of the head 210 from the bearing 208. As shown in Figures 28a and 28b, the surface 209 can have a profile which varies with respect to the bearing side 239 or the coupling surface 238.

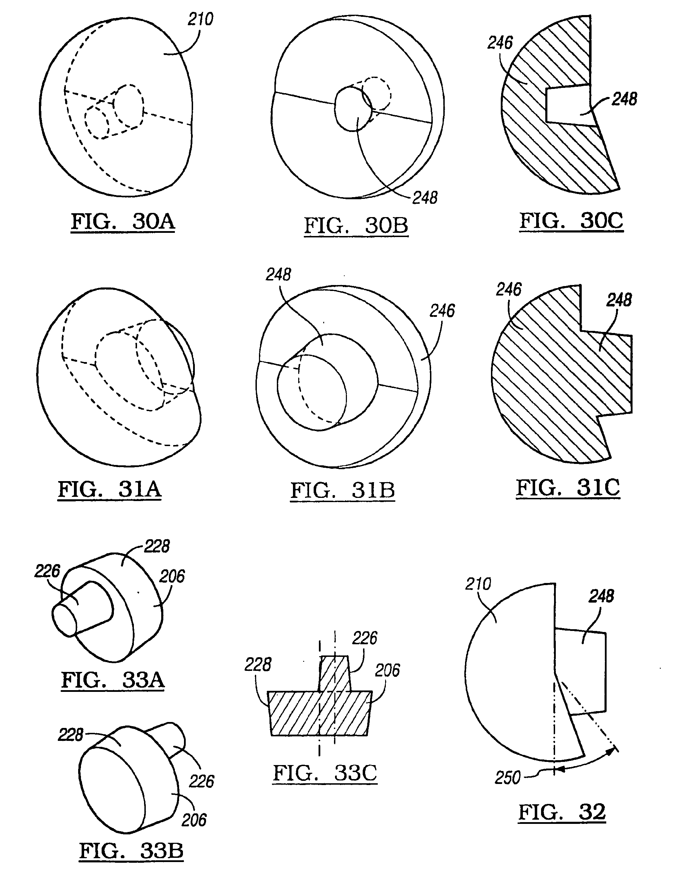

Figures 30a-32 represent alternate heads 210 that can optionally be used in the shoulder prosthetic 200. Disposed on a coupling side of the head 210 is a fixation member 248 which can be a female or male coupling taper. As shown in Figure 32, the head 210 can further have an extended articulating surface 250 which can vary in radius of curvature and in length.

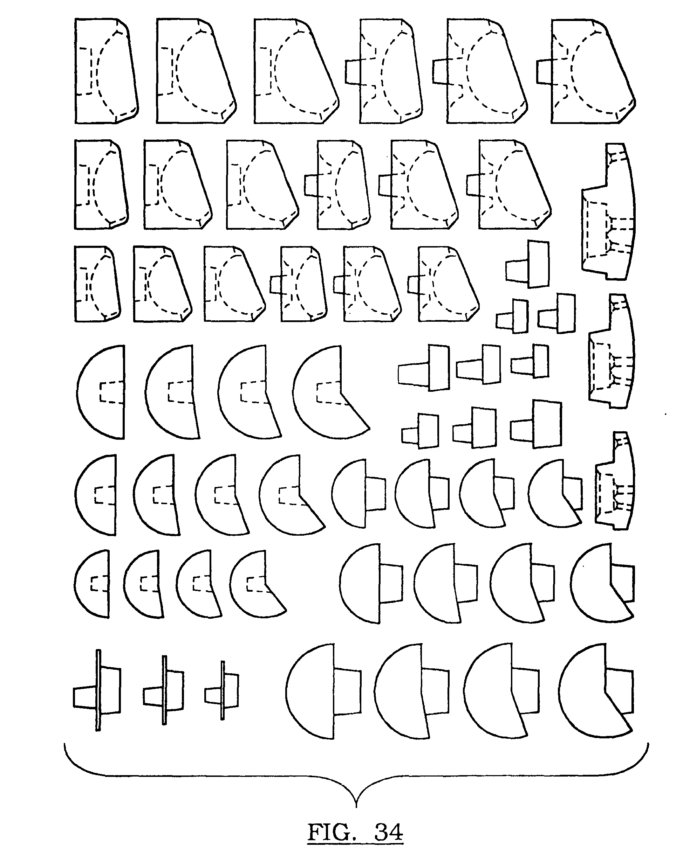

Figures 33a-33c represent adaptors 206 having varying offsets for adjusting the location of the head or bearing member within the joint. It is envisioned the adaptors 206 additionally can have varying heights which allow for varying displacement of the head from the glenoid. Figure 34 represents the kit of bearing, head, and adaptor members utilized to construct the humeral prosthetic. It is envisioned that this kit can additionally have varying stems and fixation devices such as screws.

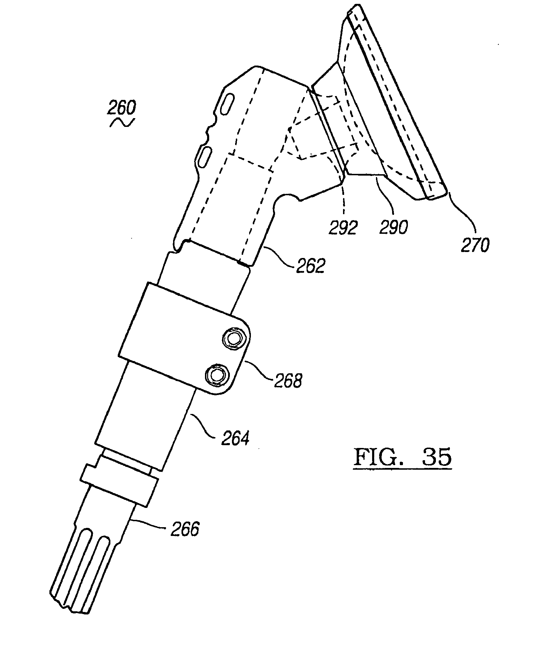

Figure 35 represents an alternate reverse humeral prosthetic 260 for use with a large segment humeral resection. The modular humeral prosthetic 260 has a humeral body portion 262, a base member 264, and a fixation stem 266. Optionally, the base member can annularly support a soft tissue fixation member 268. The humeral body portion 262 has a concave bearing surface 270 configured to articulate with a head member in a reverse shoulder. It is envisioned that this concave bearing surface 270 can be a bio-compatible polymer, metal or ceramic: The concave bearing can be coupled to a modular bearing head 290 which is mated with a coupling taper 292 defined in the humeral body portion 262.

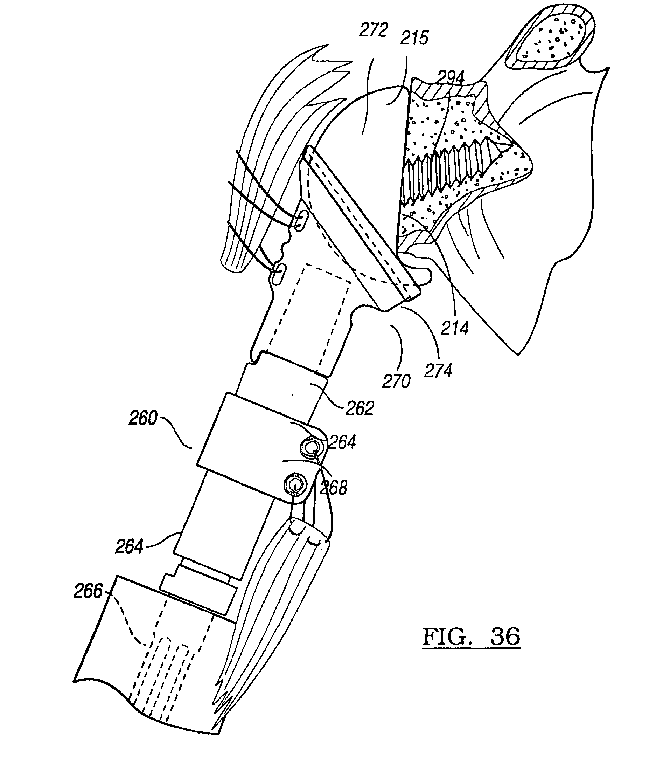

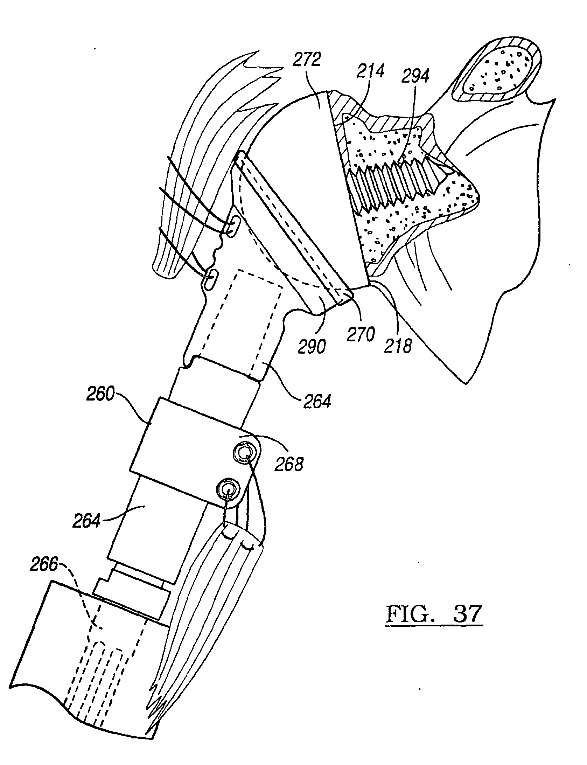

Figures 36-38 depict a side view of an implanted humeral prosthetic 260. Each humeral prosthetic 260 is mated with a head 272 that is coupled to the prepared glenoid 214. As shown in Figure 36, the head 272 of the reverse shoulder prosthetic can be coupled to the preared glenoid 214 using a large mating screw 294. Additionally, the head 272 can have extended articulating surface 274 to allow for proper articulation of the joint. As Figures 36 and 37 represent, the head 272 can have a coupling surface 215 which is generally perpendicular or angled to the fixation screw 294.

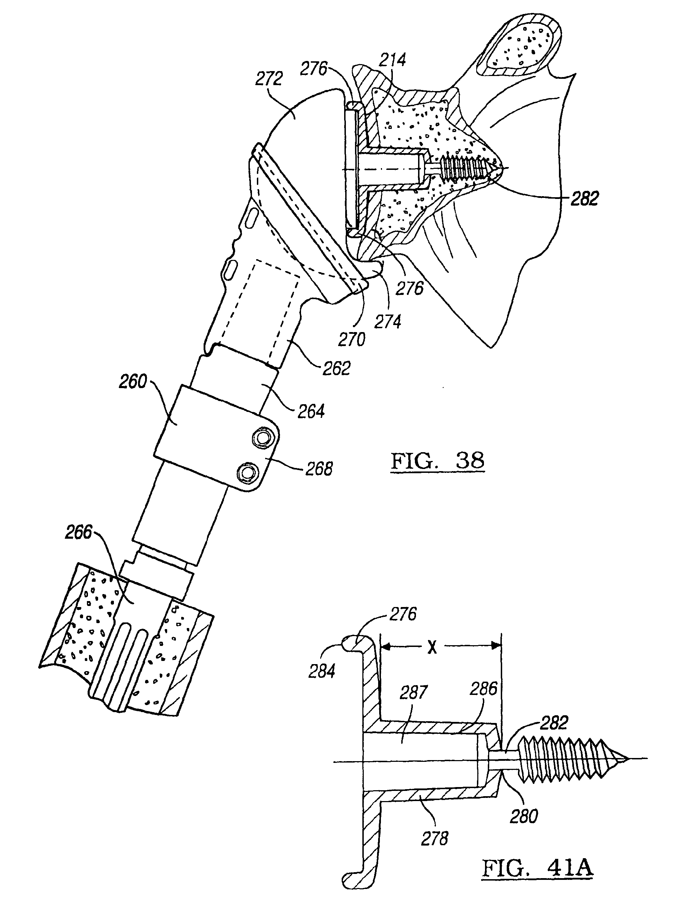

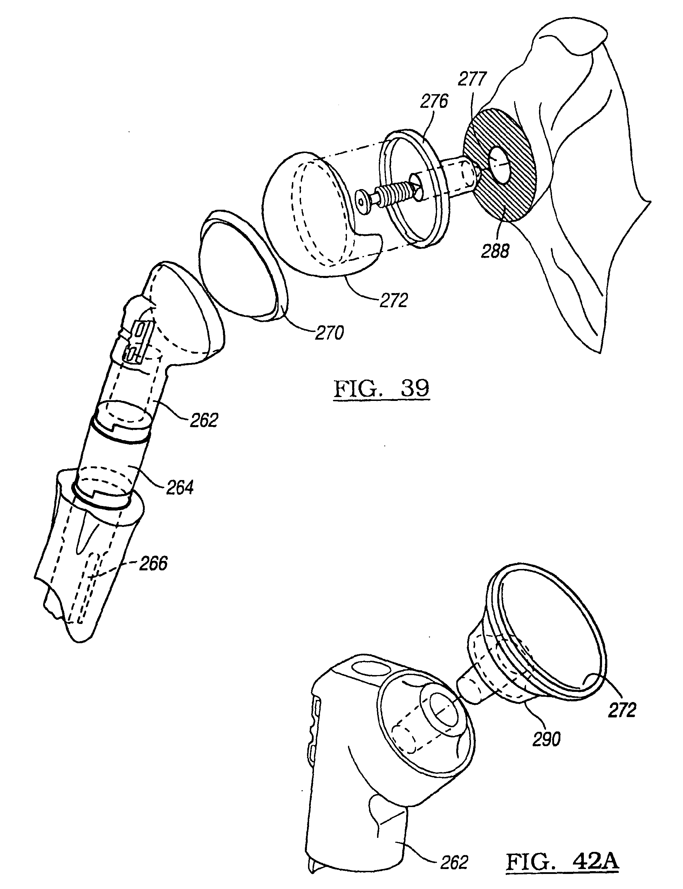

Figures 38 and 39 represent the coupling of the head portion 272 of the prosthetic to the glenoid 214. Shown is the tray 276 which is inserted into an aperture 277 defined within a coupled glenoid 288. Next, a fixation screw is used to couple the tray 276 to the prepared glenoid 288. A head 272 is couply oriented and then snapped or fixed to the tray 276.

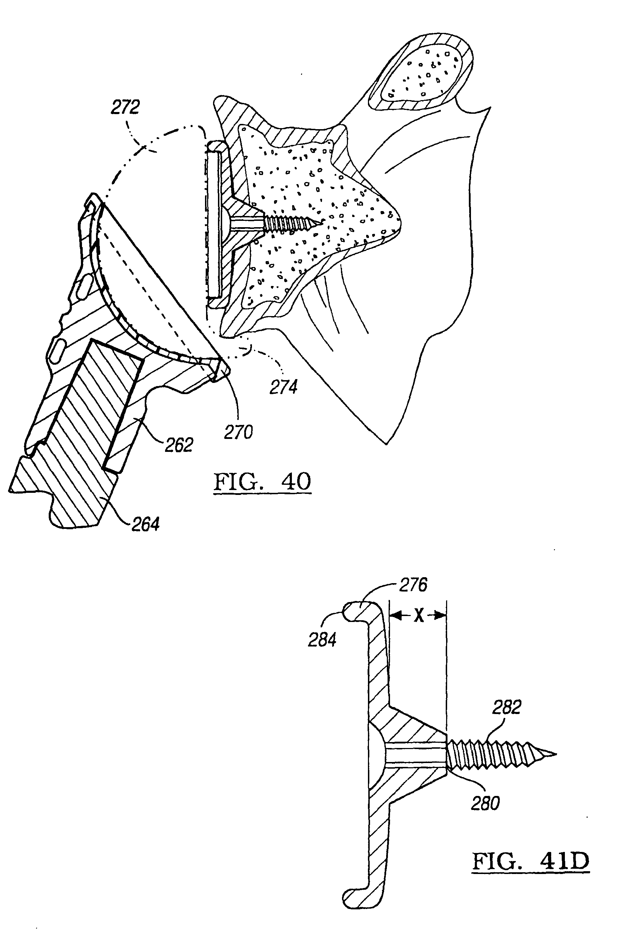

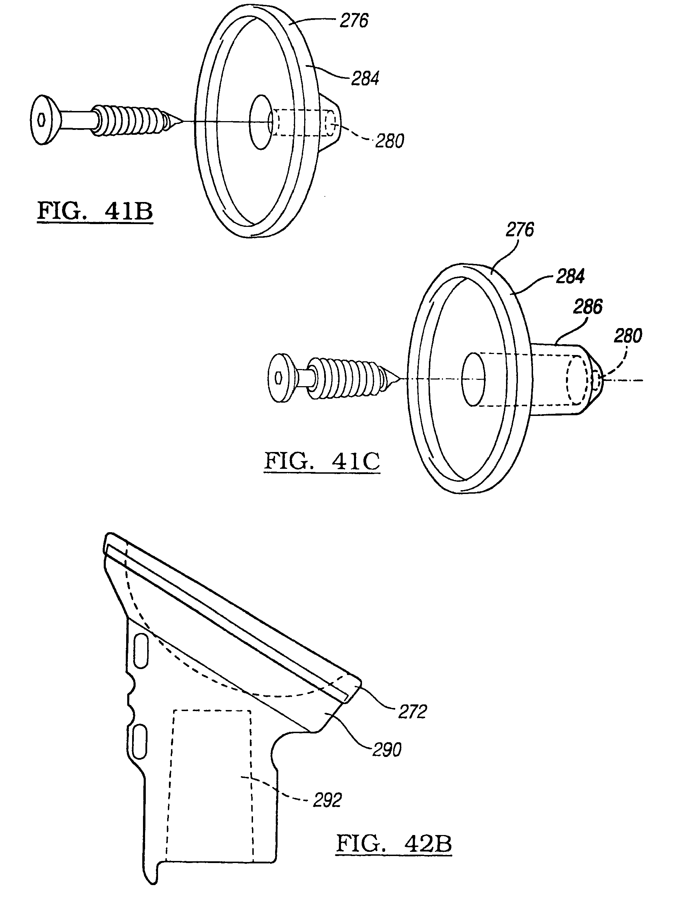

As shown in Figures 41b-41d, the tray 276 has a retaining flange 284 and a bone screw accepting aperture 280. Disposed on a coupling surface of the tray 276 is a non-threaded extended region 286 which measures in length greater than 6 millimeters. It has been found that having the extended portion 286 having a length of greater than 6 millimeters allows for the proper coupling of the tray 276 without stress concentration failures in the mating fastener. As shown in Figures 41 a and 41 b, the extended portion 286 can have a counter bore 287, which is configured to accept a head of the bone engaging fastener 282.

Figures 42a and 42b represent the humeral body portion 262. As previously mentioned, the humeral body portion 262 has a concave bearing member 272. The concave bearing member 272 can be integral with the monolithic humeral body portion 262 or may be coupled to a bearing member 290. In this regard, the bearing member 290 can be coupled to a fixation member 294 within the humeral body portion 262. The humeral body portion 262 further has a locking member 292 which can either be a male or female taper to couple the humeral body portion 262 to the base member 264.

While the invention has been described in the specification and illustrated in the drawings with reference to a preferred embodiment, it will be understood by those skilled in the art that various changes may be made and equivalents may be substituted for elements thereof without departing from the scope of the invention as defined in the claims. In addition, many modifications may be made to adapt a particular situation or material to the teachings of the invention without departing from the essential scope thereof. Therefore, it is intended that the invention not be limited to the particular embodiments illustrated by the drawings and described in the specification as the best modes presently contemplated for carrying out this invention, but that the invention will include any embodiments falling within the foregoing description and the appended claims.

## Claims

1. An implant assembly for a shoulder joint having a humerus and a glenoid, the assembly comprising:
a head bounded by a convex surface and a base, the base including a first coupling mechanism configured to mate the head to the glenoid;
a humeral stem configured to be inserted in the humerus;
a cup attachable to the stem and having a concave surface configured to articulate with the convex surface of the head, and a second adaptor disposed between the humeral stem and the cup; and
a first adaptor configured to allow displacement of at least one of the head and the cup with respect to the stem.

2. The assembly of Claim 1, wherein the first adaptor comprises a pair of offset tapers.

3. The assembly of Claim 2, wherein the cup includes a coupling feature configured to couple with a second adaptor.

4. The assembly of Claim 3, wherein the second adaptor defines a third coupling feature.

5. The assembly of Claim 1 further comprising a glenoid fixation component.

6. The assembly of Claim 5, wherein the glenoid fixation component comprises a fixation taper.

7. The assembly of Claim 1, wherein the convex surface of the head is hemispherical.

8. The assembly of Claim 1, wherein the humeral stem and the cup comprise a monolithic component.

9. The assembly of Claim 1, wherein the humeral stem is attached to the cup with a coupling taper.

10. The assembly of Claim 1, wherein the humeral stem comprises a humeral body portion, a body portion and a fixation stem.

11. A method for selectively performing reverse and traditional arthroplasty for a shoulder joint that includes a humerus and a glenoid, the method comprising:
providing a head, a cup and a humeral stem;
providing a first adaptor with an offset coupling mechanism configured to attach the head to the glenoid in reverse arthroplasty;
coupling the first adaptor to a glenoid;
coupling the cup to one of the humeral stem or the first adaptor.

12. The method according to Claim 11 further comprising providing a second adaptor configured to couple the head to the humeral stem in traditional arthroplasty.

13. The method of Claim 12, wherein the first and second comprise an offset coupling mechanism.

14. The method of Claim 12, wherein the first adaptor includes a first male taper selectively attachable to one of the humeral stem and a glenoid stem, and a second male taper configured to be received in a female taper of the head.

15. The method of Claim 14, wherein the first and second male tapers are offset with respect to each other.

16. The method of Claim 12, wherein performing the corresponding arthroplasty includes:
attaching the head to the first adaptor;
attaching the cup to the humeral stem; and
articulating the head with the cup.

17. The method of Claim 11, wherein performing the corresponding arthroplasty includes:
attaching the head to the humeral stem;
attaching the cup to the glenoid; and
articulating the head with the cup.

18. The method of Claim 16, wherein attaching the glenoid adaptor to the glenoid includes screwing a plurality of bone fixation screws through a plurality of holes defined in the glenoid adaptor.

19. An assembly of implant components for a shoulder joint having a humerus and a glenoid, the assembly comprising:
a humeral stem;
a head having a convex surface;
a cup configured to articulate with the convex surface of the head; and
an adaptor selected from the group consisting of a glenoid adaptor for a reverse shoulder arthroplasty, and a humeral adaptor for traditional shoulder arthroplasty, wherein the glenoid adaptor is configured to connect the head to the glenoid when the cup is connected to the humeral stem, and wherein the humeral adaptor is configured to connect the head to the humeral stem when the cup is connected to the glenoid, said adaptor having offset coupling tapers configured to allow relative displacement between the head and the glenoid.

20. The assembly of Claim 19, wherein the humeral adaptor is connected to the head with mating male and female tapers.

21. The assembly of Claim 19, wherein the humeral adaptor is connected to the humeral stem with mating male and female tapers.

22. The assembly of Claim 19, wherein the glenoid adaptor is connected to the head with mating male and female tapers.

23. The assembly of Claim 19, wherein the glenoid adaptor is connected to the glenoid using a plurality of bone fixation screws.

24. An assembly of implant components for a shoulder joint having a humerus and a glenoid, the assembly comprising:
a plurality of humeral stems;
a plurality of heads;
a plurality of cups configured to articulate with corresponding heads;
a plurality of offset glenoid adaptors for a reverse shoulder arthroplasty, and a plurality of humeral adaptors for traditional shoulder arthroplasty, wherein each offset glenoid adaptor is configured to connect one of the heads to the glenoid when one of the cups is connected to one of the humeral stems, and wherein each humeral adaptor is configured to connect one of the heads to one of the humeral stems when one of the cups is connected to the glenoid.

25. The assembly of Claim 24 wherein at least one humeral adaptor comprises a pair of offset tapers.

26. The assembly of Claim 24 wherein at least one glenoid adaptor comprises a pair of offset tapers.

27. The assembly of Claim 26, wherein said modular glenoid adaptor defines a convex coupling surface.

28. The assembly of Claim 24, wherein at least one cup is modular.

29. The assembly of Claim 28, wherein said modular cup includes a bearing base and a bearing.

30. The assembly of Claim 24, wherein the plurality of humeral stems includes humeral stems of different sizes.

31. The assembly of Claim 24, wherein the plurality of heads includes heads of different sizes.

32. The assembly of Claim 24, wherein the plurality of cups includes cups of different sizes.

33. The assembly of Claim 24, wherein the plurality of glenoid adaptors includes glenoid adaptors of different sizes.

34. The assembly of Claim 24, wherein the plurality of humeral adaptors includes humeral adaptors of different sizes.

35. The assembly of Claim 34, wherein some glenoid adaptors are also humeral adaptors.

36. The assembly of Claim 24, wherein some of the glenoid adaptors are connected to corresponding heads with mating male and female tapers.

37. The assembly of Claim 36, wherein said glenoid adaptors are connected to corresponding glenoid stems with other mating male and female tapers.

38. The assembly of Claim 24, wherein some of the humeral adaptors are connected to corresponding heads with mating male and female tapers.

39. The assembly of Claim 38, wherein said humeral adaptors are connected to corresponding humeral stems with other mating male and female tapers.
